Europäisches Patentamt

(19) European Patent Office   (11) Publication number:   **0 103 887**

Office européen des brevets   **A2**

(12) **EUROPEAN PATENT APPLICATION**

(21) Application number: 83109332.3

(22) Date of filing: 20.09.83

(51) Int. Cl.³: **C 12 N 15/00**
**C 12 P 17/02, C 12 P 7/22**
**C 12 P 7/24, C 12 P 7/42**

(30) Priority: 20.09.82 US 419953

(43) Date of publication of application:
28.03.84 Bulletin 84/13

(84) Designated Contracting States:
AT BE CH DE FR GB IT LI LU NL SE

(71) Applicant: Amgen
1900 Oak Terrace Lane
Thousand Oaks California 91320(US)

(72) Inventor: Ensely, Burt D.
4002 Calle Mira Monte
Newsbury Park California 91320(US)

(74) Representative: Brown, John David et al,
FORRESTER & BOEHMERT Widenmayerstrasse 4/I
D-8000 München 22(DE)

(54) Method and materials for the microbiological oxidation of aromatic hydrocarbons.

(57) Disclosed are novel DNA sequences susceptible to expression in a microorganism in the form of synthesis of one or more enzymes participative in the microbiological oxidative degradation of a selected aromatic hydrocarbon substrate to a selected oxidized compound which is an intermediate in a multiple enzyme-catalyzed degradative pathway for total mineralization of said substrate. The DNA sequences are characterized by being substantially free from operative association with DNA sequences susceptible to expression in the form of synthesis of one or more enzymes participative in oxidative degradation of said selected intermediate compound. Illustrative of the invention is a plasmidborne DNA sequence of *Pseudomonas putida* origin which codes for expression, in a host microorganism such as *E. coli*, of enzymes participative in the oxidative degradation of naphthalene to salicylate. Cultured growth of stably transformed host cells in a medium including a naphthalene substrate results in quantitative naphthalene degradation and accumulation of isolatable quantities of, e.g., salicylate products.

EP 0 103 887 A2

## BACKGROUND

The present invention relates generally to hydrocarbon bioconversion and, more specifically, to the use of recombinant DNA techniques to confer upon microorganisms the capacity for selective partial oxidative degradation of aromatic hydrocarbons with the consequent accumulation of desired oxidized compounds in high yield.

While microbial metabolic processes have been employed since virtually prehistoric times in such valuable agriculturally-related endeavors as the making of alcoholic beverages, the leavening of bread, the production of vinegar, cheese and butter, and the retting of flax, it has only been with the advent of the modern science of microbiology in the nineteenth century that the capacity of microorganisms for effecting the rapid synthesis and transformation of organic compounds has begun to be fully exploited. In the pharmaceutical industries microorganisms are now widely employed in such diverse processes as the production of antibiotics and the selective oxidative transformation of steroid hormones into highly biologically active forms. Industrial chemical uses of microorganisms have been even more varied, ranging from the use of large, mixed-species cultures of organisms in waste treatment to the use of highly specialized single strains of organisms for the synthesis of proteinaceous enzymes, organic acids (including amino acids) and complex carbohydrates such as xantham gum. See, e.g., U.S. Letters Patent Nos. 4,275,163; 4,286,060; and 4,218,538.

New strains of organisms are constantly being isolated, new cell-free microbial enzyme systems are being characterized and new mutant strains of organisms are being developed by chemical mutagens and radiation processing -- all with the goal of effecting industrial scale transformations of hydrocarbons, especially oxidative transformations for which certain organisms are particularly well suited. See, e.g., U.S. Letters Patent Nos. 4,250,259; 4,241,184; 4,269,940; 4,266,034; and 4,268,630.

Some substantial research and development efforts have been directed to the use of microbial systems in the oxidative transformation of naphthalene to salicylate. Briefly summarized, the discovery in the early 1950's that salicylate and other naphthalene dissimilation products could be isolated from selected pseudomonal cultures spurred numerous efforts to develop fermentation conditions which would optimize salicylic acid accumulation. See, e.g., Holser, Biotechnology and Biogengineering, Vol. V., pp. 243-251 (1963); Ishikura, et al. Agr.Biol.Chem., 32, pp. 12-20 (1968); Zajic, et al., U.S. Letters Patent No. 3,274,074; and Indian Patent No. 130541 (Gazette of Indian, May 3, 1975, page 281). No substantial commercial implementation of the fermentation processes disclosed in these studies has been effected.

Genetic engineering techniques involving transduction, cell fusion and conjugation have been extensively explored in the development of strains of microorganisms capable of effecting the total oxidative degradation ("mineralization") of complex hydrocarbons such as are found in petroleum spills and industrial wastes. See, e.g., U.S. Letters Patent No. 3,813,316 and 4,259,444. See, also, Chatterjee, et al., "Plasmids in the Biodegradation of Chlorinated Aromatic Compounds", pp. 519-528 in Molecular Biology, Pathogenicity, and Ecology of Bacterial Plasmids, Levy, et al., eds. Plenum Pub. Corp. (1981). The observation that genes coding for hydrocarbon oxidation enzymes are, in some soil organisms, carried in clusters or families on transmissible plasmids was in great part responsible for much of this successful conjugation and plasmid-assisted molecular breeding work.

The development of genetic engineering methods employing recombinant DNA techniques for molecular cloning of DNA segments has been widely heralded as providing an entire new basis for the industrial development of microorganisms suitable for even more varied forms of hydrocarbon synthesis and transformation. See, e.g., U.S. Letters Patent No. 4,237,244; 4,264,731; and 4,278,765. The state of the art with respect to the potential for use of recombinant DNA techniques to expand and enhance the utilization of microorganisms in the area of hydrocarbon oxidation was extensively treated by Shapiro, et al. in "Perspectives for Genetic Engineering of Hydrocarbon Oxidizing Bacteria", Trends in the Biology of Fermentation for Fuels and Chemicals, Hollaender, ed., Plenum Press (1981). Briefly summarized, the Shapiro, et al. publication treats: (1) the nature of hydrocarbon substrates susceptible to microbial oxidation including unoxidized hydrocarbons generally, and linear, cyclic and substituted hydrocarbons in particular; (2) the state of knowledge with regard to oxidative pathways for hydrocarbon breakdown; (3) the known degradative DNA plasmids of Pseudomonas and related bacteria; (4) genetic analysis of known multiple enzyme catalyzed hydrocarbon oxidation pathways; and (5) the general prospects for the genetic engineering of hydrocarbon oxidizing bacteria. Shapiro, et al. conclude that, while the prospects for genetic engineering of hydrocarbon oxidizing bacteria are good, "What distinguishes this kind of genetic engineering from that used to produce single proteins for bacterial cells is the need to construct cells with coordinated sets of sequential biochemical activities. This will require more than wizardry with restriction endonucleases and synthetic DNA fragments, important

- 4 -          0103887

as these tools are. Genetic engineering of hydrocarbon oxidizers will also require knowledge of the physiology of different pathways and a background in the genetic analysis of each one".

Of particular interest to the background of the present invention are published studies treating the nature and characteristics of transmissible, plasmid-coded hydrocarbon oxidizing enzymes, especially those involved with aromatic hydrocarbon mineralization. The work of D. T. Gibson and his co-workers has been especially significant and includes: Gibson, <u>Arch. Biochem. & Biophys.</u>, <u>142</u>, pp. 394-396 (1971); Jeffrey, et al., <u>Biochemistry</u>, <u>14</u>, pp. 575-584 (1975); Gibson, "Microbial Degradation of Polycyclic Aromatic Hydrocarbons", pp. 57-66 in <u>Proc. Third Int'l. Biodegradation Symposium</u>, Sharpley, et al., eds., Applied Sciences Publishers, Ltd., London (1975); Gibson, "Microbial Transformations of Aromatic Pollutants", pp. 187-204 in <u>Aquatic Pollutants, Transformation and Biological Effects</u>, Hutzinger, et al., eds. Pergamon Press (1977); and, Gibson, "Microbial Metabolism" appearing at pp. 161-192 in "Handbook of Environmental Chemistry" Vol. II, Part A, Hutzinger, ed., Springer-Verlag, Heidelberg (1980). In a like manner, the work of E. A. Barnsley and his co-workers [appearing, e.g., in <u>J. Bacteriology</u>, <u>125</u>, pp. 404-408 (1976) and in Connors, et al. <u>J. Bacteriology</u>, <u>144</u>, pp. 1096-1101 (1982)] has also served to illuminate the processes involved in oxidative degradation of aromatic compounds. See also, Davies, et al., <u>Biochem. Journal</u>, <u>91</u>, p. 251 (1964) and Cain, "Transformation of Aromatic Hydrocarbons" appearing at pp. 99-131 in "Hydrocarbons in Biotechnology" Harrison, et al., eds. Heydon & Son, Ltd., London (1980). The Cain reference provides a fairly comprehensive review of attempts to optimize bioconversion processing of aromatic hydrocarbons by means of media modification, mutant formation, genetic manipulations, development

- 5 -

0103887

of co-oxidation systems, use of enzyme inhibitors, use of substituted substrate analogs and various other regulatory procedures.

As an example of the most recent work in this area, the applicant and his co-workers provided the results of an exhaustive study into the oxidation of naphthalene by a multicomponent enzyme system from Pseudomonas sp. NC1B 9816 [See, Ensley, et al., J. Bacteriology, 149, pp. 948-954 (1982)] and therein characterized with specificity the initial reaction in naphthalene oxidation, resolving the naphthalene dioxygenase enzyme(s) involved into three protein components. In companion studies reported in the doctoral dissertation of Alice A. Laborde, The University of Texas at Austin (University of Texas Library, reference No. L113) the substrate specificity of strain NC1B 9816 for a wide variety of substituted and unsubstituted aromatic compounds is reported. The work done with this strain is intimately correlated to the studies of I. C. Gunsalus and his co-workers concerning transmissible plasmids coding for early enzymes of naphthalene oxidation in Pseudomonas putida. See, e.g., Catteral, et al., J. General Microbiol., 67, pp. 117-124 (1971); Dunn, et al., J. Bacteriology, 114, pp. 974-979 (1973) and the very recent studies on gene organization of the "Nah 7" plasmid appearing in Yen, et al. P.N.A.S. U.S.A., 79, pp. 874-878 (1982).

In the last-mentioned study, determinations of gene organization for two gene clusters coding for degradation of naphthalene and salicylate substrates were carried out through use of polar mutations involving insertion of a selected transposon (Tn5, coding for a kanamycin resistant phenotype). As a result of the transposon insertion, mutant plasmids were formed which included the total, multi-enzyme coding gene clusters for total mineralization but had full operation of the clusters interrupted in a manner allowing for mapping of gene functions on the basis of accumulation of oxidized intermediate compounds formed, activities of selected enzymes, and ascertainment of the site of transposon insertion. While DNA plasmids such as formed in the course of these studies are extremely valuable as research tools for developing an understanding of the relationship between enzymes generated and oxidative degradation intermediates formed upon gene expression, they have limited potential for application in industrial processes for microbial manufacture of desired intermediates in a total hydrocarbon oxidation pathway toward microbial mineralization of a given substrate. This is so because these mutant plasmids share with naturally-occurring and artificially-formed mutants a residual operative association with plasmid-borne or genomic genetic materials (i.e., DNA sequences) which code for enzymes participative in the degradation of the pathway intermediates. Simply put, at any time in the continued cultured growth of mutant organisms or organisms harboring mutant plasmids, the natural capacity of the cells to repair interruptive mutations (with or without direct involvement of chromosomal genes) can be expected to result in reversion to the original phenotype, i.e., reversion to the capacity for total mineralization of the substrate through all intermediate steps.

At present, therefore, and despite substantial advances having been made in the understanding of the physiology and genetics of microbial oxidation of aromatic hydrocarbons, the art has not been provided with any description of methods and materials for the certain and continuous production by microorganisms of desired oxidized aromatic compounds from selected aromatic hydrocarbon chemical substrates.

## BRIEF SUMMARY

The present invention provides, in one of its aspects, a novel class of DNA sequences derived by recombinant DNA techniques and useful in effecting partial microbiological oxidative degradation of selected aromatic hydrocarbon substrates with resultant accumulation of desired oxidized aromatic compounds. The procedures by which the novel DNA sequences of the invention are generally obtained initially involve isolating and amplifying continuous DNA sequences (e.g., gene clusters) susceptible to coordinated microbial expression in the form of synthesis of a plurality of enzymes participative in a degradative, multiple enzyme-catalyzed pathway for the mineralization of a selected substrate. While preferred sources of DNA sequence starting materials are existing degradative plasmids [including the known transmissible, pseudomonad-borne naphthalene (Nah), toluene (TOL), salicylate (SAL), and xylene (XYL)-degrading plasmids], genomic DNA and cDNA replicas derived from genomic DNA may also be employed. The isolated and amplified gene clusters are treated with restriction endonuclease enzymes to secure DNA fragments which: (a) code for microbial expression of enzymes participative in oxidative degradation of the selected substrate to a selected intermediate within a total mineralization pathway; and (b) are physically isolated from, and hence free of operative association with, sequences coding for the degradation of the selected intermediate.

DNA sequence products of the invention are inserted in a suitable, autonomously-replicating expression vector and vectors so formed are employed in the transformation of host cells. Cells transformed with the novel vectors are multiplied and disposed in a medium comprising the selected aromatic hydrocarbon substate, whereupon cellular enzymes effect the partial degradation of the substrate with consequent accumulation of the desired intermediate substance.

In one of its presently most preferred forms, the present invention provides novel DNA sequences of Pseudomonas putida origin which: (a) are capable of microbial expression in the form of synthesis of one or more selected enzymes participative in a portion of a total enzymatic pathway for mineralization by oxidative degradation of naphthalene; and (b) are free of operative association with DNA sequences coding for enzymes participative in the further degradation of selected oxidized naphthalene degradation products formed due to the presence of the selected enzymes. More specifically provided are DNA sequences derived from the Pseudomonas putida plasmid Nah 7 which code for synthesis in E. coli of naphthalene dioxygenase and, optionally in sequence, one or more of the following enzymes: cis-naphthalene-1,2-dihydrodiol dehydrogenase; dihydroxy naphthalene dioxygenase; hydroxychromene carboxylate isomerase; hydroxybenzalpyruvate aldolase; and salicylaldehyde dehydrogenase.

As a consequence of the preparation of such novel DNA sequences and the incorporation of same into a suitable DNA expression vector, transformed host microbial cells may be disposed in a medium including naphthalene and will therein degrade the naphthalene to one or more of the following specifically selected products: cis-1,2-naphthalene dihydrodiol; 1,2-dihydroxy naphthalene (isolated in the cellularly-autooxidized from of naphthoquinone); hydroxychromene carboxylate; hydroxybenzalpyruvate; salicylaldehyde; and/or salicylate. Products isolated from the cells or their supportive medium may be suitably purified and put to use in precisely the manner of chemically-synthesized compounds, e.g., as pharmaceuticals or reagent intermediates in the production of other desired compounds. The isolation of cis-1,2-naphthalene dihydrodiol [more

specifically, 1(S), 2(R)-dihydroxy-1,2-dihydronaphthalene] through practice of the invention will, for example, provide an appropriate feedstock for acid treatment in the commercial formation of 1-naphthol.

Further aspects and advantages of the present invention will become apparent upon consideration of the following detailed description of presently preferred embodiments thereof.

## DETAILED DESCRIPTION

The methods and materials which provide an illustration of the practice of the invention and which comprise the presently preferred embodiment relate specifically to plasmid-borne DNA sequences of Pseudomonas putida origin which can be employed to transform a desired host microbial species (such as E. coli). Cells transformed according to this embodiment express the DNA sequences in the form of synthesis of an initial, naphthalene dioxygenase enzyme or enzyme system alone or in combination with one or more enzymes sequentially participative in a complex multiple-enzyme-catalyzed degradation pathway from naphthalene to salicylate. Transformation vectors of the invention and transformed host cells do not include DNA sequences which are susceptible to expression as enzymes coding for degradation of salicylate or, optionally, any other desired intermediate compound in the naphthalene to salicylate pathway.

The following illustrative examples treat: (1) the selective isolation from the P. putida naphthalene mineralization plasmid Nah 7 of a relatively large DNA fragment including sequences coding for the entire naphthalene to salicylate sequence of oxidative degradation enzymes; (2) the determination that a transformed E. coli strain including the isolated fragment is capable of degrading naphthalene through salicylate and not beyond, presumptively due to the presence of all sequentially operative necessary enzymes for this transformation and the absence of expression of genes coding for salicylate degradative enzymes; (3) a procedure employed to identify the salicylate metabolite formed by transformed host cells from the naphthalene substrate and determine the rate of its formation; (4) the isolation and incorporation into E. coli expression vectors of smaller, Nah operon-containing DNA fragments which retain the capacity to direct naphthalene to salicylate degradation upon expression in transformed E. coli host cells; (5) the incorporation of a naphthalene to salicylate degradative DNA sequence into a broad host range expression vector; and (6) development of further expression vectors according to the invention.

## EXAMPLE 1

Plasmid pNUT, which codes for the expression of Pseudomonas putida hydrocarbon degradative enzymes in an Escherichia coli host, was constructed by the following procedure. The plasmid Nah 7 [Yen and Gunsalus, P.N.A.S. U.S.A., 79, pp. 874-878 (1982)] was isolated from Pseudomonas putida pPG 7 by an alkaline-SDS procedure of Hansen and Olsen, J. Bacteriol., 135, pp. 227-238 (1978) and digested to completion with Hind III. The digested Nah 7 DNA was mixed with Hind III-digested pBR322 (A.T.C.C. 37017) and incubated with DNA ligase. The ligated DNA was transformed into E. coli HB101 and the transformed cells were plated onto L-agar containing 200 µg/ml ampicillin. The plates were incubated for 24 hr. and the resultant colonies were exposed to the vapors of naphthalene crystals placed in the lid of the plate. A single colony, designated strain 625, turned brown after exposure to naphthalene vapors for 24-48 hr. Formation of the brown color indicated that strain 625 was producing one or more metabolites from naphthalene since naphthoquinone, a dark brown compound, is a by-product of naphthalene metabolism in Pseudomonas species [Shamsuzzamon and Barnsley, Biochem. Biophys. Res. Comm., 60, pp. 582-589 (1974) and Davies, et al., supra]. Plamsid pNUT was isolated from strain 625. This plasmid was approximately 21,000 base pairs (21Kb) in size and produced fragments of 4.4Kb and 16.5Kb in size after digestion with Hind III. The 4.4Kb Hind III fragment had the same mobility during agarose gel electrophoresis and the same pattern of restriction enzyme fragmentation as pBR322, while the large 16.5Kb fragment was presumed to be a portion of the Nah 7 plasmid, which carried genes coding for the synthesis of naphthalene degradative enzymes.

## EXAMPLE 2

E. coli strain 625 was analyzed for naphthalene metabolism by measuring the accumulation of non-volatile metabolites from $^{14}$C-labelled naphthalene [Ensley, et al., J. Bacteriol., 149, pp. 949-954 (1982)]. E. coli strain 625 was transferred from an isolated colony to 1.0 ml of L-broth containing a 200 µg/ml ampicillin and incubated in a 13 x 100 mm test tube with shaking at 30°C until the cultures had reached an optical density at 600 nm ($OD_{600}$) of 1.0-2.0. The cells were pelleted in a centrifuge at 5,000 x g for 10 min. The cell pellet was suspended in 50 mM sodium phosphate, pH 7.0 to give a cell suspension with a measured $OD_{600}$ of 0.25. A 0.5 ml portion of the cell suspension was mixed with 5.0 µl of dimethylformamide containing 50 n moles of [$^{14}$C] naphthalene (specific activity 2.5 mCi/m mol) and incubated with shaking for 30-60 min. at 30°C. The reaction was terminated by transferring a 20 µl aliquot of the reaction mixture to the surface of a 1.5 x 2 cm square of pre-coated thin layer chromatography sheet. The spotted sheet was dried under a stream of air for 15 min., which removed unused naphthalene, and non-volatile [$^{14}$C] naphthalene metabolites on the sheet were detected by placing the sheet in 7.0 ml of liquid scintillation counting cocktail and measuring residual radioactivity in a liquid scintillation counter. Strain 625 produced measurable levels of [$^{14}$C] naphthalene metabolites with this procedure, while no naphthalene metabolism could be detected when untransformed E. coli HB101, or HB101 transformed with pBR322, were analyzed.

The rate of naphthalene degradation by E. coli strain 625 was measured with a modification of the method described above. The cell suspension containing labelled naphthalene was sampled after 10, 20, 30 and 40 minutes of incubation, and the amount of non-volatile naphthalene metabolites produced after each time interval was determined by liquid scintillation counting. A linear increase in the formation of naphthalene metabolites over time was observed in this experiment. The results are shown in Table 1, infra.

## EXAMPLE 3

The metabolite(s) produced from [$^{14}$C] naphthalene by E. coli strain 625 were identified by the following procedure. A cell suspension containing [$^{14}$C] naphthalene as described in Example 2 was incubated for 1-12 hr. The suspension was then extracted twice with 0.5 ml volumes of ethyl acetate. The organic phases were removed and combined (neutral extract), and the cell suspension was acidified by the addition of 50 µl of 1.0 M HCl. The acidified suspension was extracted twice with 0.5 ml volumes of ethyl acetate, and the organic phases were removed and combined (acid extract). The neutral and acid organic extracts were reduced to dryness under vacuum and the residues dissolved in 50 µl of ethyl acetate. A 10 µl portion of each extract was analyzed by liquid scintillation counting to determine the amount of [$^{14}$C] naphthalene metabolites which could be extracted under neutral and acidic conditions. The remainder of the extracts was applied separately to the origin of a pre-coated thin layer chromatography (TLC) sheet. In addition, standards of salicylate, salicylaldehyde, α naphthol, naphthoquinone, and cis-1,2-naphthalene dihydrodiol in ethyl acetate solutions were applied in separate spots to the sheet. The chromatogram was developed in a solvent system of chloroform/methanol/acetic acid, 40:1:2 (vol/vol). The developed chromatogram was viewed under UV light and the locations of the standards were marked. The chromatogram was then exposed to Kodak AR4 X-ray film for 24 hr and the film developed to identify the location of the [$^{14}$C] naphthalene metabolites. Over 90% of the naphthalene metabolites produced by E. coli strain 625 were extractable in ethyl acetate only after acidification of the cell suspension, indicating the production of an acidic metabolite. Analysis of the metabolites by TLC and autoradiography revealed that strain 625 produces, from naphthalene, a single major metabolite which is acidic and has the same relative mobility during chromatography as that of authentic salicylic acid. This data indicated that E. coli strain 625 carries a plasmid containing that fragment of Nah 7 DNA which codes for the synthesis of enzymes involved in the oxidation of naphthalene to salicylic acid. This fragment thus comprises at least part of the gene sequence described as the Nah operon (Yen and Gunsalus, supra), i.e., sequences coding for the three protein 1,2-naphthalene dioxygenase system, cis-1,2-dihydrodiol dehydrogenase, dihydroxy naphthalene dioxygenase, hydroxychromene carboxylate isomerase, hydroxy benzalpyruvate aldolase, and salicylaldehyde dehydrogenase. Plasmid pNUT thus includes specific DNA sequences coding for degradative enzymes which are free from operative association with sequences coding for salicylate hydroxylase or similar sequences coding for the degradation of salicylate.

### EXAMPLE 4

Plasmids pE3 and pE317, which are smaller than pNUT but still carry the genes associated with the Nah operon, were constructed by the following procedure. Plasmid pNUT DNA was digested with Eco RI, which produced 5 fragments visible after agarose gel electrophoresis. The digested DNA was re-ligated to produce a population of plasmid DNA's containing a variable number of the 5 Eco RI fragments. The ligated DNA was transformed into E. coli HB101 and brown colonies were selected and scored as described in Example 1. One clone carried a new plasmid, designated pE3, which contained only 2 Eco RI sites and was 17.3 Kb in size. The plasmid was isolated and again digested with Eco RI. The large Eco RI fragment (10.9Kb) resulting from this digestion was purified by agarose gel electrophoresis and recovered from the agarose gel with NA-45 DEAE membranes (Schleicher and Schuell, Inc., Keene, NH) used according to the manufacturer's recommendations. The purified Eco RI fragment was ligated into Eco RI-cut pBR322 DNA and transformed into E. coli HB101. Recombinants were selected and scored as described in Example 1. Several clones carried a new plasmid which contained pBR322 (4.4 Kb) and a 10.9Kb insert in the Eco RI site. This plasmid (15.3Kb) was designated pE317. E. coli strains carrying plasmids pE3 and pE317 were analyzed for rates and products of naphthalene metabolism (Table 1, infra). Both plasmids coded for the synthesis of enzymes necessary for the metabolism of naphthalene to salicylic acid, and were expressed at detectable levels in an E. coli host.

EXAMPLE 5

The Nah operon genes on the 10.9Kb Eco RI fragment described in Example 4 were cloned into a braod host-range vector by the following procedure. The 10.9Kb Eco RI fragment described in Example 4 was ligated to Eco RI cut R1162, a cloning vector with broad host-range properties [Meyer, et al., J. Bacteriol., 150, pp. 552-562 (1982)]. The ligated DNA was transformed into E. coli HB101 and recombinant cells were selected and scored as described in Example 1. A new plasmid, designated R3174, was isolated from these clones and contained the 10.9Kb insert in the Eco RI site of R1162. E. coli transformed with R3174 was analyzed for rates and products of naphthalene metabolism as described in Examples 2 and 3 (Table 1). The results of this experiment demonstrate that the essential gene of the Nah operon can be transferred to a cloning vector which is stably maintained in a wide range of bacterial hosts.

TABLE 1

Rates and Products of Naphthalene Metabolism
By E. Coli HB101 Containing Recombinant Plasmids

| Example | Plasmid | Vector | Size | Oxidation Product[a] | Rate[b] |
|---------|---------|--------|------|----------------------|---------|
| 2 | pNUT | pBR322 | 21Kb | Salicylate | 5.2 |
| 4 | pE3 | pBR322 | 17.3Kb | Salicylate | 43.4 |
| 4 | pE317 | pBR322 | 15.3Kb | Salicylate | 44.0 |
| 5 | R3174 | R1162 | 21Kb | Salicylate | 45.8 |

[a] Product formed through the oxidation of naphthalene

[b] Rate of product formation in nmoles/hr/ml of reaction mixture as described in Example 3.

In a study conducted to ascertain the rate of expression of plasmid R3174 when transformed into a Pseudomonas putida strain C1 a spontaneous transformation of the plasmid into a 13.5Kb form was observed. The resultant plasmid, designated pCR4, retained its capacity to degrade naphthalene rather rapidly but could not process intermediates in the naphthalene to salicylate pathway beyond the formation of dihydroxy naphthalene as evidenced by the accumulation of naphthoquinone in the cells and the absence of ascertainable quantities of the products of operation of dihydroxy naphthalene dioxygenase enzyme, when analyzed by the procedures described in Examples 2 and 3.

Further studies were conducted to determine the degree to which the 10.9 Kb DNA sequence of Nah 7 origin and maintained on plasmid pE317 of Example 4 could be shortened and still retain: (1) the capacity to oxidize naphthalene to any given oxidized product; and (2) the capacity to oxidize naphthalene to salicylate. The results of these studies are reported in Example 6.

## EXAMPLE 6

Digestion of plasmid pE317 with XhoI results in the formation of 2 restriction fragments, a small fragment of approximately 2.5 Kb in size and a large 12.8 Kb fragment which contains all of pBR322 and 8.4 Kb of the Nah 7 DNA. Plasmid pX1 was constructed by digesting pE317 with XhoI and separating the 2 fragments by agarose gel electrophoresis. The large XhoI fragment was recovered from the gel as described in Example 4, ligated to itself, and transformed into E. coli HB101. Transformed cells were detected by plating onto L-agar containing 200 μg/ml ampicillin. Colonies which grew on these plates were analyzed for the presence of plasmid DNA and were found to carry a plasmid of 12.8 Kb in size containing a single XhoI restriction site. Plasmid pXI displayed patterns of restriction digestion consistent with the indication that this plasmid is formed from pE317 after deletion of the 2.5 Kb XhoI fragment near the center of the Nah 7 insert present in pE317 (Table III).

Plasmid p5H was constructed by digesting pE317 DNA with PvuII, which produced 5 fragments of various sizes. The digested DNA was re-ligated to produce a population of plasmid DNAs as described in Example 4. The ligated DNA was transformed into E. coli HB101 and brown colonies were selected and scored as described in Example 1. One clone contained a plasmid, designated p5H, which produced only 2 PvuII restriction fragments after PvuII digestion and agarose gel electrophoresis. Plasmid p5H is approximately 11.2 Kb in size and subsequent analysis as described in Examples 2 and 3 revealed that this deletion product of pE317 carries some of the gene sequence described as the Nah operon.

Studies of E. coli HB101 transformed with pXI revealed no substantial degradation of naphthalene at all over a period of 24 hours. Cells similarly transformed with p5B retain the capacity to degrade naphthalene at a much slower rate than pE317-transformed cells and the degradation products formed have been tentatively identified as hydroxybenzylpyruvate.

A study was made of the rates of naphthalene oxidation of all the above-constructed plasmids in specified transformed hosts as compared to the rate of oxidation of the Nah 7 plasmid in Pseudomonos sp. NCIB 9816, strain 11 and the results of the study are set out in Table II below. The plasmid selected for comparative use included the entire cluster of naphthalene dissimilative genes but had the cis-dihydrodiol dehydrogenase enzyme gene inactivated by a deletion mutation with the functional result of accumulation of dihydrodiol products. In this table, therefore, the rate of oxidation of naphthalene to dihydrodiol product by the mutant Nah plasmid-containing P. species (i.e., 240 nanomoles/hr/ml) is assigned a standard value of 100 and absolute oxidation rates of the remaining cell types are designated in relationship to this standard.

## TABLE II

| Plasmid | Replicon | Host | Size | Detected Oxidation Product | Rate |
|---------|----------|------|------|----------------------------|------|
| pE317 | pBR322 | HB101 | 15.3Kb | Salicylate | 18.1 |
| pE713* | pBR322 | HB101 | 15.3Kb | Salicylate | 8.3 |
| p5H | pBR322 | HB101 | 11.2Kb | Hydroxyben-zalpyruvate (tentative) | 3.7 |
| pXI | pBR322 | HB101 | 13 Kb | None | 0 |
| pR3174 | pR1162 | HB101 | 27 Kb | Salicylate | 19.1 |
| pCR4 | pR1162 | PpC1 | 13.6Kb | Naphtho-quinone | 27.6 |
| Nah | Nah | Pseudo-monas sp. NCIB 9816 strain 11 | 83 Kb | Naphthalene dihydrodiol | 100 |

_____

\* Same as p317 but with Nah 7 plasmid-origin DNA in reverse orientation.

Plasmids pE317, pR3174, p5H and pCR 4 were subjected to restriction endonuclease fragmentation studies with various common endonucleases and the results of these studies are set out in approximately-scaled, semi-graphic form in Table III below.

## TABLE III

### pE317

pBR322

| | |
|---|---|
| amp → | Eco RI |
| | Bam HI |
| | Hind III |
| | Pst |
| | Cla I |
| | Sal I |
| | Xho I |
| | Sma I |
| | Bgl II |
| | Pvu II |

### p5H

pBR322

| | |
|---|---|
| amp → | Eco RI |
| | Pvu II |
| | Bgl II |
| | Sal I |
| | Pst I |
| | Bam HI |
| | Xho I |
| | Sma I |

### PR3174

pR1162

| | |
|---|---|
| | Eco RI |
| | Sal I |
| | Xho I |
| | Bgl II |
| | Sma I |
| | Bam HI |

### pCR4

pR1162

| | |
|---|---|
| | Eco RI |
| | Xho I |
| | Sma I |
| | Bam HI |
| | Sal I |

Table IV below recapitulates a portion of the DNA sequencing information of Table III for plasmid pE317 with a graphic representation of the likely positions on the P. putida-derived fragment of genes or gene systems coding for the following enzymes:

A.  Naphthalene dioxygenase
B.  Cis-1,2-dihydrodiol dehydrogenase
C.  1,2-Dihydroxy naphthalene dioxygenase
D.  Hydrochromene carboxlate isomerase
E.  Hydroxybenzalpyruvate aldolase
F.  Salicylaldehyde dehydrogenase.

### TABLE IV

pE317

| A | B | C | D | E | F |

pBR322

| Position | | | | | | | Enzyme |
|---|---|---|---|---|---|---|---|
| I | I | | | | | I | Eco RI |
| | I | | I | I | | | Bam HI |
| | I | | I | | I | | Sal I |
| | | I | I | | | | Xho I |
| | | I | | | | | Sma I |
| | | | I | | | | Bgl II |
| I | I | | | I | I | | Pvu II |

It will be apparent from the above that the formation of p5H from pE317 involved deletion of Pvu II-Pvu II fragments both in the portion of the plasmid which was of pBR322 origin as well as in the portion which appears to code for enzymes E and F. Plasmid pCR4, on the other hand, appears to have undergone deletion of at least that part of the fragment coding for enzyme C which includes the unique BglII site but not to have suffered deletion of that portion including the two Xho I sites associated with the DNA sequences coding for enzymes A and B. Loss of naphthalene oxidation activity by plasmid pXI (as noted above) is consistent with deletion of an essential portion of the A and B enzyme regions upon Xho I digestion.

It will be apparent from the above that appropriately ordered restriction endonuclease treatment of plasmid pE317 will result in the generation of plasmid-borne DNA sequences coding for enzyme A alone or enzyme A in combination with sequences coding for enzyme B, or both enzymes B and C, or enzymes B, C and D, or enzymes B, C, D, and E, allowing for the selective formation of the entire range of intermediates in the oxidative degradation of naphthalene to salicylate.

Once the Nah operon genes of interest have been cloned into a high-copy number plasmid under control of a high efficiency, artificially regulated (temperature, external inducer, etc.) promoter system, the following fermentation schemes are anticipated.

1. Cells will be grown in a simple medium to an appropriate density with the Nah-regulating promoter turned off (little or no Nah enzyme synthesis). This will result in rapidly growing cells with enhanced genetic stability.

2. The culture will be treated in a manner which causes the Nah-regulating promoter to be turned on, resulting in synthesis of high levels of the Nah enzymes. The culture will then be transferred to a medium which will not support rapid growth (to reduce demand of the culture for oxygen).

The "poised" culture will be mixed with naphthalene under high aeration and allowed to accumulate oxidation products from naphthalene.

Since naphthalene oxidation products have been observed to have toxic properties (Cain reference, supra), some provisions will be made to remove the oxidation product as it is formed. There can include the addition of adsorbents such as activated charcoal, ion exchange resins (Tone, et al., Biotechnol. Bioeng., 10, 689 (1968)), or boron compounds (Zajic, et al., U.S. Patent No. 3,274,074) or the use of techniques such as dialysis culture (Abbott, et al., Biotechnol. Bioeng., 12, 577-589 (1970)).

While the foregoing illustrative examples all relate to methods and materials useful in microbial degradation of naphthalene to selected intermediates in the multiple enzyme-catalyzed oxidative degradation to a mineralized state, the invention is applicable to numerous other sequential enzyme systems and associated pathways for a variety of aromatic hydrocarbons.

As previously noted, the aromatic hydrocarbon dissimilative operons (gene clusters) presently available in the form of the pseudomonad-borne plasmids TOL, SAL, XYL, and the like, are also expected to provide appropriate DNA starting materials for practice of the invention. Manipulations of these plasmids in the manner described above with regard to the Nah 7 plasmid will similarly generate DNA sequences susceptible to microbial expression in the form of synthesis of one or more enzymes participative in oxidative degradation of selected aromatic hydrocarbons (e.g., toluene, salicylate, xylene) to selected oxidized products in isolatable quantities. In this regard, DNA sequence products of such manipulations will all be characterized by the freedom from operative association with DNA sequences coding for degradation of the desired "intermediate" oxidized products.

In the foregoing illustrative examples relating to naphthalene oxidizing enzymes, expression of genes or gene clusters coding for the enzymes remained under the control of their "original" promoter/regulator DNA sequences, i.e., those controlling expression of the enzymes in the pseudomonal cells from which the plasmid DNA was obtained. It is certainly within the contemplation of the present invention that the novel DNA sequences may be provided, according to well-known methods, with different control DNA regions allowing for optimal expression in selected host cells.

Numerous other modifications and variations of the invention as above-described are expected to occur to those skilled in the art and consequently only such limitations as appear in the appended claims should be placed thereon.

0103887

WHAT IS CLAIMED IS:

1. A DNA sequence susceptible to expression in a microorganism in the form of synthesis of one or more enzymes participative in the microbiological oxidative degradation of a selected aromatic hydrocarbon substrate to a selected oxidized compound which is an intermediate in a multiple enzyme-catalyzed microbial degradative pathway for total mineralization of said substrate, said DNA sequence characterized by being substantially free from operative association with DNA sequences susceptible to microbial expression in the form of synthesis of one or more enzymes participative in degradation of said selected intermediate compound.

2. A DNA sequence according to claim 1 which is duplicative of a portion of a DNA sequence present in a pseudomonad-borne DNA plasmid specifying synthesis of a plurality of enzymes sequentially participative in the total mineralization of an aroamtic hydrocarbon selected from the group consisting of naphthalene, xylene, toluene and salicylate.

3. A DNA sequence according to claim 2 which is duplicative of a DNA sequence present in an Nah plasmid specifying synthesis of a plurality of enzymes sequentially participative in the total mineralization of naphthalene.

4. The sequence according to claim 1 wherein said selected aromatic hydrocarbon substrate is naphthalene and said selected intermediate compound is salicylate.

5.   The sequence according to claim 1 wherein said selected aromatic hydrocarbon substrate is naphthalene and said selected intermediate compound is salicylaldehyde.

6.   The sequence according to claim 1 wherein said selected aromatic hydrocarbon substrate is naphthalene and said selected intermediate compound is hydroxybenzalpyruvate.

7.   The sequence according to claim 1 wherein said selected aromatic hydrocarbon substrate is naphthalene and said selected intermediate compound is hydroxychromene carboxylate.

8.   The sequence according to claim 1 wherein said selected aromatic hydrocarbon substrate is naphthalene and said selected intermediate compound is 1,2-dihydroxy naphthalene.

9.   The sequence according to claim 1 wherein said selected aromatic hydrocarbon substrate is naphthalene and said selected intermediate compound is cis-1,2-naphthalene dihydrodiol.

10.   A process for effecting microbiological oxidative degradation of a selected aromatic hydrocarbon substrate to a selected oxidized compound which is an intermediate in a multiple enzyme-catalyzed microbial degradative pathway for total mineralization of said substrate comprising:

(1) transforming a microorganism which is incapable of oxidative degradation of said selected intermediate compound with a transformation vector comprising a DNA sequence according to claim 1;

(2) disposing microorganisms transformed according to step (1) in a medium comprising said selected aromatic hydrocarbon substrate; and,

(3) isolating said selected intermediate compound from said microorganisms.

11.  A process according to claim 10 wherein said DNA sequence is duplicative of a portion of a DNA sequence present in a pseudomonad-borne DNA plasmid specifying synthesis of a plurality of enzymes sequentially participative in the total mineralization of an aroamtic hydrocarbon selected from the group consisting of naphthalene, xylene, toluene and salicylate.

12.  A process according to claim 11 wherein said DNA sequence is duplicative of a DNA sequence present in an Nah plasmid specifying synthesis of a plurality of enzymes sequentially participative in the total mineralization of naphthalene.

13.  A process according to claim 10 wherein said selected aromatic hydrocarbon substrate is naphthalene and the selected intermediate compound isolated is salicylate.

14.  A process according to claim 10 wherein said selected aromatic hydrocarbon substrate is naphthalene and the selected intermediate compound isolated is salicylaldehyde.

15.  A process according to claim 10 wherein said selected aromatic hydrocarbon substrate is naphthalene and the selected intermediate compound isolated is hydroxybenzalpyruvate.

16. A process according to claim 10 wherein said selected aromatic hydrocarbon substrate is naphthalene and the selected intermediate compound isolated is hydroxychromene carboxylate.

17. A process according to claim 10 wherein said selected aromatic hydrocarbon substrate is naphthalene and the selected intermediate compound isolated is naphthoquinone, the product of microbial autooxidation of 1,2-dihydroxy naphthalene.

18. A process according to claim 10 wherein said selected aromatic hydrocarbon substrate is naphthalene and the selected intermediate compound isolated is cis-1,2-naphthalene dihydrodiol.

19. A process for preparing the DNA sequence for use in effecting quantitative microbiological oxidative degradation of a selected aromatic hydrocarbon substrate to a selected oxidized compound which is an intermediate in a multiple enzyme-catalyzed microbial degradative pathway for total mineralization of said substrate, said process comprising:
isolating a DNA sequence susceptible to expression in a microorganism in the form of synthesis of a plurality enzymes participative in a degradative multiple enzyme catalyzed pathway for total mineralization of a selected aromatic hydrocarbon substrate; and
selectively deleting from said DNA sequence a portion of said sequence susceptible to expression in the form of synthesis of one or more enzymes participative in oxidative degradation of a selected oxidized compound which is an intermediate in said pathway for total mineralization of said substrate.

20. The process according to claim 19 wherein said DNA sequence is duplicative of a portion of a DNA sequence present in a pseudomonad-borne DNA plasmid specifying synthesis of a plurality of enzymes sequentially participative in the total mineralization of an aromatic hydrocarbon selected from the group consisting of naphthalene, xylene, toluene and salicylate.

21. The process according to claim 20 wherein said DNA sequence is duplicative of a DNA sequence present in an Nah plasmid specifying synthesis of a plurality of enzymes sequentially participative in the total mineralization of naphthalene.

22. The process according to claim 19 wherein said selected aromatic hydrocarbon substrate is naphthalene and said selected intermediate compound is salicylate.

23. The process according to claim 19 wherein said selected aromatic hydrocarbon substrate is naphthalene and said selected intermediate compound is salicylaldehyde.

24. The process according to claim 19 wherein said selected aromatic hydrocarbon substrate is naphthalene and said selected intermediate compound is hydroxybenzalpyruvate.

25. The process according to claim 19 wherein said selected aromatic hydrocarbon substrate is naphthalene and said selected intermediate compound is hydroxychromene carboxylate.

26. The process according to claim 19 wherein said selected aromatic hydrocarbon substrate is naphthalene and said selected intermediate compound is 1,2-dihydroxy naphthalene.

27. The process according to claim 19 wherein said selected aromatic hydrocarbon substrate is naphthalene and said selected intermediate compound is cis-1,2-naphthalene dihydrodiol.

28. The process of claim 19 further including the step of inserting the resultant DNA sequence into a DNA transformation vector.